# EUROPEAN PATENT APPLICATION

(11) **EP 2 030 677 A2**
(43) Date of publication of application: **04.03.2009**
(21) Application number: 08015319.0
(22) Date of filing: 29.08.2008
(51) Int. Cl.: B01J 19/00, C40B 50/18, G01N 33/543

(54) **Biosensor chip, process for producting the same, and sensor for surface plasmon resonance analysis**

(30) Priority: 29.08.2007 JP 2007222065
(71) Applicant: FUJIFILM Corporation, Minato-ku Tokyo (JP)
(72) Inventor: Takeuchi, Yohsuke, Ashigarakami-gun Kanagawa-ken (JP); Nishimi, Taisei, Ashigarakami-gun Kanagawa-ken (JP); Tsuzuki, Hirohiko, Ashigarakami-gun Kanagawa-ken (JP)
(74) Representative: HOFFMANN EITLE

(57) **Abstract**

A biosensor chip includes a substrate, a polymer having an anionic functional group and being arranged on a surface of the substrate, a polyamino group which is directly or indirectly bound to the anionic functional group at a surface of the polymer, and a long-chain alkyl-based group which is directly or indirectly bound to the anionic functional group at the surface of the polymer. Therefore, the biosensor chip can immobilize a bioactive material on the surface of the biosensor chip without influence of electric charge repulsion.

## Description

### BACKGROUND OF THE INVENTION

### Field of the Invention

The present invention relates to a biosensor chip which can usefully immobilize a bioactive substance, and a process for producing the biosensor chip. The present invention also relates to a sensor for surface plasmon resonance analysis.

### Description of the Related Art

Currently, measurement performed by utilizing interaction between molecules such as immunoreaction is frequently carried out, for example, for clinical inspection. Especially, several techniques enabling highly sensitive detection of variations in the amount of a material to be detected without bothersome operation or use of labelled material are currently in practical use. For example, the SPR (surface plasmon resonance) measurement technique, the QCM (quartz crystal microbalance) measurement technique, the technique using the functionalized surfaces of gold particles having various dimensions in the range from colloidal particles to ultrafine particles, or other techniques are currently used.

In the measurement chips for use in measurement in which the surface plasmon resonance is utilized, an evaporated metal film and a thin film having a functional group are formed in this order on a transparent substrate (e.g., a glass substrate), where the functional group can immobilize a bioactive substance such as a protein, and the bioactive substance is immobilized on the surface of the metal film through the functional group. Therefore, it is possible to analyze interaction between biomolecules by measurement of a specific binding reaction between the above bioactive substance and a specimen material.

The above-mentioned bioactive substance is a biological macromolecule which is a basic component of a living body (such as a nucleic acid, a protein, or a polysaccharide), or a constituent element of a biological macromolecule (such as a nucleotide, a nucleoside, an amino acid, or a type of sugar), or a material which controls a living body or changes a function of a living body (such as a lipid, a vitamin, and a hormone). The bioactive substance is important, for example, in development of medicine components and functional foods.

The lipids are materials which have in a molecule a long-chain fatty acid or a similar hydrocarbon chain, and have various functions. For example, the lipids may become an energy source or a membrane constituent molecule, or may participate in signal propagation in a cell or nucleus.

Although the lipids can be classified into the simple lipids, the complex lipids, and the lipid derivatives, especially, the phospholipids and the glycolipids are receiving attention in connection with carbohydrate metabolism. Although the phospholipids are insoluble in water, the phospholipids form micelles which are insoluble in water since the phospholipids have a molecular structure containing a polar group and a nonpolar group and being amphiphilic. The phosphoric acid in a phospholipid is hydrophilic. Therefore, when a phospholipid comes in contact with a solvent, the phospholipid can form a liposome (a lipid membrane vesicle), which is a soluble micelle formed with an artificial phospholipid membrane.

Since the phospholipids have the above property, the phospholipids are currently being used in study of a biomembrane model, and the use of the phospholipids as materials in drug delivery systems are currently proceeding toward commercialization and are being actively studied.

For example, the Biacore L1 chip is known as a chip which can trap lipids, liposomes, and the like. In the Biacore L1 chip, dextran with which a substrate is coated is modified with a long-chain alkane, so that lipid liposomes and the like can be absorbed by the long-chain alkane. (Biacore is a trademark of GE Healthcare Companies.) See M.A. Cooper et al., "A Vesicle Capture Sensor Chip for Kinetic Analysis of Interactions with Membrane-Bound Receptors," Analytical Biochemistry, Vol. 277, Issue 2, pp. 196-205, 2000, and E.M. Erb et al., "Characterization of the Surfaces Generated by Liposome Binding to the Modified Dextran Matrix of a Surface Plasmon Resonance Sensor Chip," Analytical Biochemistry, Vol. 280, Issue 1, pp. 29-35, 2000.

However, since the Biacore L1 chip has the carboxyl group, which is anionic, the Biacore L1 chip cannot absorb anionic or nonionic lipids or liposomes although the Biacore L1 chip can efficiently absorb cationic lipids or liposomes.

### SUMMARY OF THE INVENTION

The present invention has been made in view of the above circumstances.

The first object of the present invention is to provide a biosensor chip which can immobilize a bioactive substance on a surface of the biosensor chip without influence of electric charge repulsion.

The second object of the present invention is to provide a process for producing a biosensor chip which achieves the first object.

The third object of the present invention is to provide a sensor for surface plasmon resonance analysis, where the sensor uses the biosensor chip which achieves the first object.
(I) In order to accomplish the first object, according to the first aspect of the present invention, a biosensor chip is provided. The biosensor chip according to the first aspect of the present invention comprises a substrate; a polymer having an anionic functional group and being arranged on a surface of the substrate; a first polyamino group which is directly or indirectly bound to the anionic functional group at a surface of the polymer; and a long-chain alkyl-based group which is directly or indirectly bound to the anionic functional group at the surface of the polymer.
   The expression "directly bound to the to the anionic functional group" means to be directly bound to the anionic functional group without a linking group, and the expression "indirectly bound to the anionic functional group" means to be indirectly bound to the anionic functional group through a linking group. The linking group may be derived from the polymer, or may be bound to the polymer for binding the first polyamino group or the long-chain alkyl-based group to the polymer.
   The long-chain alkyl-based group is preferably an alkyl chain containing 10 to 22 carbon atoms, and more preferably an alkyl chain containing 12 to 18 carbon atoms. In some cases, one or more heteroatoms may exist between sequences of carbon atoms constituting the long-chain alkyl-based group, and/or one or more double bonds and/or one or more triple bonds may exist between single-bonded sequences of carbon atoms constituting the long-chain alkyl-based group. In addition, it is preferable that the long-chain alkyl-based group have a normal-chain structure (which is not branched).
   The anionic functional group is typically the carboxyl group.
   In addition, preferably, the biosensor chip according to the first aspect of the present invention may further have one or any possible combination of the following additional features (i) to (vii).
   (i) It is preferable that the first polyamino group have an acyl group at an end of the first polyamino group.
   (ii) The long-chain alkyl-based group may be bound to the first polyamino group. In this case, the biosensor chip may further comprise a second polyamino group which is directly or indirectly bound to the polymer.
   (iii) It is preferable that the first polyamino group be a diaminoalkylene group or a di(aminoalkyl) ether group.
   (iv) It is preferable that the long-chain alkyl-based group is an alkyl-based group having 10 to 22 carbon atoms.
   (v) It is preferable that the polymer is carboxymethyl dextran.
   (vi) It is preferable that the polymer is arranged on the substrate through a metal film.
   (vii) It is preferable that the metal film is composed of at least one of the metals of gold, silver, copper, platinum, and aluminum.
(II) In order to accomplish the second object, according to the second aspect of the present invention, a process for producing a biosensor chip is provided. The process according to the second aspect of the present invention comprises the steps of: (a) activating an anionic functional group in a polymer arranged on a surface of a substrate; and (b) binding a first compound containing a polyamino group to the anionic functional group, and thereafter binding a second compound containing a long-chain alkyl-based group to the anionic functional group.
(III) In order to accomplish the third object, according to the third aspect of the present invention, a sensor for surface plasmon resonance analysis comprising the biosensor chip according to the first aspect of the present invention is provided. That is, the biosensor chip according to the first aspect of the present invention can be preferably used as a sensor chip in a sensor for surface plasmon resonance analysis.
(IV) The advantages of the present invention are explained below.

Since the biosensor chip according to the first aspect of the present invention comprises the polymer having an anionic functional group (e.g., the carboxyl group) and being arranged on a surface of the substrate, and a polyamino group directly or indirectly bound to the anionic functional group at a surface of the polymer, and a long-chain alkyl-based group directly or indirectly bound to the anionic functional group at the surface of the polymer, the long-chain alkyl-based group can stably immobilize a bioactive substance on the surface of polymer, and the polyamino group can cancel the electric charge of the anionic functional group in the polymer. Therefore, the biosensor chip according to the first aspect of the present invention can immobilize the bioactive substance on the surface of the polymer without influence of the electric charge repulsion. In addition, although the nonspecific absorption can be frequently caused by electric charge, the biosensor chip according to the first aspect of the present invention can reduce the nonspecific absorption while the bioactive substance is immobilized.

### BRIEF DESCRIPTION OF THE DRAWINGS

FIG. 1 is a diagram schematically illustrating an arrangement of chemical components in a first embodiment of the biosensor chip according to the present invention.
FIG. 2 is a diagram schematically illustrating an arrangement of chemical components in a second embodiment of the biosensor chip according to the present invention.
FIG. 3 is a diagram schematically illustrating an arrangement of chemical components in a third embodiment of the biosensor chip according to the present invention.
FIG. 4 is a diagram schematically illustrating a configuration of a sensor for surface plasmon resonance analysis, which comprises the biosensor chip according to the present invention.
FIG. 5 is a sensorgram of the biosensor chip according as a concrete example 1 of the biosensor chip according to the present invention.
FIG. 6 is a sensorgram of a conventional biosensor chip as a comparison example.

### DETAILED DESCRIPTION OF THE INVENTION

Preferred embodiments of the present invention are explained in detail below with reference to drawings.

### 1. Biosensor Chips

### 1.1 Structures of Producing Biosensor Chips

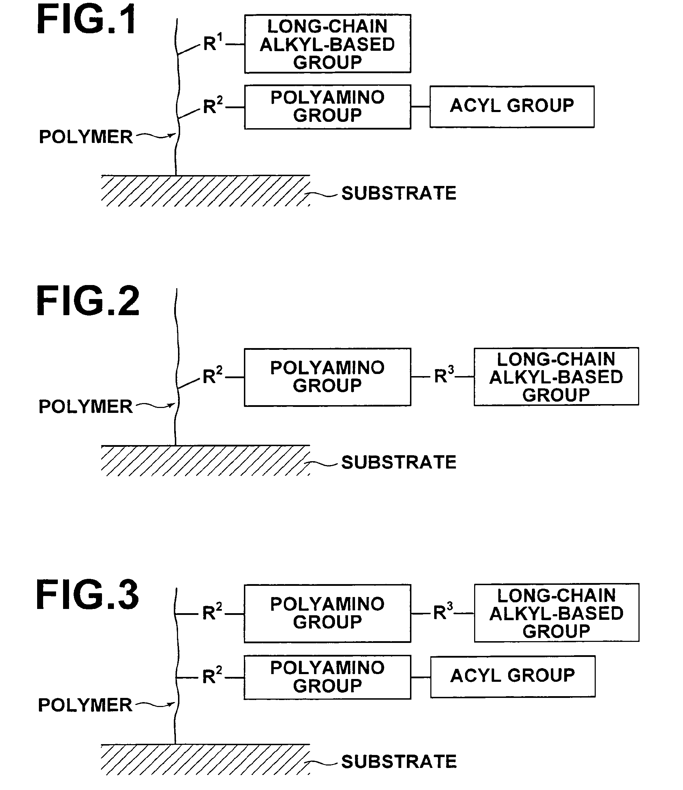

Hereinbelow, the biosensor chips according to the first to third embodiments of the present invention are explained below with reference to FIGS. 1 to 3, which schematically show the arrangements of chemical components in the first to third embodiments.

As illustrated in FIG. 1, the biosensor chip according to the first embodiment comprises a substrate, a polymer having the carboxyl group (as the aforementioned anionic functional group) and being arranged on a surface of the substrate, a long-chain alkyl-based group which is bound to the surface of the polymer through a group R¹, and a polyamino group which has an acyl group at en end of the polyamino group and is bound to the surface of the polymer through a group R². In the biosensor chip according to the first embodiment, the polyamino group can cancel the electric charge of the carboxyl group in the polymer, and the long-chain alkyl-based group can stably immobilize a bioactive substance on the surface of polymer.

As illustrated in FIG. 2, the biosensor chip according to the second embodiment comprises a substrate, a polymer having the carboxyl group (as the aforementioned anionic functional group) and being arranged on a surface of the substrate, a polyamino group which is bound to the surface of the polymer through the group R², and a long-chain alkyl-based group which is bound to the polyamino group through a group R³. In the biosensor chip according to the second embodiment, the polyamino group can cancel the electric charge of the carboxyl group in the polymer, and the long-chain alkyl-based group can stably immobilize a bioactive substance on the surface of polymer.

As illustrated in FIG. 3, the biosensor chip according to the third embodiment comprises a substrate, a polymer having the carboxyl group (as the aforementioned anionic functional group) and being arranged on a surface of the substrate, a first polyamino group which is bound to the surface of the polymer through the group R², a long-chain alkyl-based group which is bound to the first polyamino group through the group R³, and a second polyamino group which has an acyl group at en end of the second polyamino group and is bound to the surface of the polymer through the group R². In the biosensor chip according to the third embodiment, the polyamino group can cancel the electric charge of the carboxyl group in the polymer, and the long-chain alkyl-based group (bound through the linking groups) can stably immobilize a bioactive substance on the surface of polymer.

As mentioned before, the biosensor chips according to the first to third embodiments basically comprise a polymer having the carboxyl group (as the aforementioned anionic functional group) and being arranged on a surface of a substrate, a polyamino group which is directly or indirectly bound to the surface of the polymer, and a long-chain alkyl-based group which is directly or indirectly bound to the surface of the polymer. Although, in the biosensor chips illustrated in FIGS. 1 to 3, the long-chain alkyl-based group is indirectly bound to the surface of the polymer through the group R¹, and the polyamino group is also indirectly bound to the surface of the polymer through the group R², the long-chain alkyl-based group or the polyamino group can be directly bound to the carboxyl group in the polymer. Therefore, the groups R¹ and R² can be dispensed with. In addition, although, in the biosensor chip illustrated in FIG. 2, the long-chain alkyl-based group is indirectly bound to the polyamino group through the group R³, the group R³ can be dispensed with in the case where one or both of the long-chain alkyl-based group and the polyamino group have a functional group behaving as a linking group which can bind the long-chain alkyl-based group and the polyamino group.

It is necessary to couple an acyl group to an open end of the polyamino group in order to suppress nonspecific absorption which can occur at the open end of the polyamino group. However, in the case where the long-chain alkyl-based group is bound to the polyamino group (as in the biosensor chips of FIGS. 2 and 3), the acyl group can be dispensed with since the long-chain alkyl-based group suppresses the nonspecific absorption.

In the biosensor chips of FIGS. 1, 2, and 3, the group R¹ is preferably the aminocarbonyl group, the carbamoyl group, the oxycarbonyl group, the carbonyloxy group, the carbonyl group, the ether group, the thioether group, or the like, the group R² is preferably the carbamoyl group, the carbonyloxy group, the carbonyl group, or the like, and the group R³ is preferably the carbamoyl group, the carbonyloxy group, the carbonyl group, or the like. Especially, from the viewpoint of the reactivity and the binding stability, it is further preferable that the group R¹ be the aminocarbonyl group or the carbamoyl group, the group R² be the carbonyl group, and the group R³ be the carbonyl group.

The long-chain alkyl-based group is preferably an alkyl chain containing 10 to 22 carbon atoms, and more preferably an alkyl chain containing 12 to 18 carbon atoms. In some cases, one or more heteroatoms may exist between sequences of carbon atoms constituting the long-chain alkyl-based group, and/or one or more double bonds and/or one or more triple bonds may exist between single-bonded sequences of carbon atoms constituting the long-chain alkyl-based group. In addition, it is preferable that the long-chain alkyl-based group have a normal-chain structure (which is not branched). Specifically, preferable examples of the long-chain alkyl-based group are the lauryl group, the myristyl group, the cetyl group, the stearyl group, the arachidyl group, the behenyl group, the oleyl group, and the like. Among all, the stearyl group and the oleyl group are particularly preferable since the stearyl group and the oleyl group can realize both of simple modification reaction and absorption of lipids.

Each atomic group realizing the polyamino group contains preferably two to four amino groups, and more preferably two or three amino groups. Specifically, the diaminoalkylene groups and the di (aminoalkyl) ether groups can be used as the polyamino group. More specifically, preferable examples of the polyamino group are the following aliphatic diamine groups, aromatic diamine groups, and polyamine groups. The aliphatic diamine groups include the ethylenediamine group, the tetraethylenediamine group, the octamethylenediamine group, the decamethylenediamine group, the piperazine group, the triethylenediamine group, the diethylenetriamine group, the triethylenetetramine group, the dihexamethylenetriamine group, the 1,4-diaminocyclohexane group, and the like. The aromatic diamine groups include the paraphenylenediamine group, the methaphenylenediamine group, the paraxylylenediamine group, the methaxylylenediamine group, the 4,4'-diaminobiphenyl group, the 4,4'-diaminodiphenylmethane group, the 4,4'-diaminodiphenylketone group, the 4,4'-diaminodiphenylsulfonic acid group, and the like. The polyamine groups include the diethylenetriamine group, the triethylenetetramine group, the tetraethylenepentamine group, the pentaethylenehexamine group, the spermidine group, the spermine group, the polyethyleneimine, and the like. Especially, from the viewpoint of water solubility and high reactivity in the modification reaction, the ethylenediamine group, the di(2-aminoethyl) ether group, and the di (3-aminopropyl) ether group can be preferably used.

The acyl group is generally expressed by the formula R⁴CO-, and the group R⁴ may be discontinued by substitution with a heteroatom in some cases. The group R4 is preferably an alkyl chain containing 1 to 21 carbon atoms, and more preferably an alkyl chain containing 1 to 11 carbon atoms. In addition, the group R⁴ preferably has a normal-chain structure (which is not branched), and may be a hydrocarbon chain containing one or more doubles bond and/or one or more triple bonds in some cases. Specifically, preferable examples of the acyl group are the formyl group, the acetyl group, the propanoyl group, the isopropanoyl group, the butanoyl group, the lauryl group, the myristyl group, the stearyl group, the oleyl group, and the like. Especially, from the viewpoint of reactivity, the formyl group, the acetyl group, and the propanoyl group can be preferably used.

### 1.2 Process for Producing Biosensor Chip

The biosensor chips according to the first to third embodiments can be produced by activating the carboxyl group (contained in the polymer arranged on the substrate) in a similar manner to the activation of a polymer bound to a substrate which is coated with a self-assembled film (as explained later), binding a compound having a polyamino group (i.e., a polyamino compound) to the activated carboxyl group, and thereafter binding a compound having a long-chain alkyl-based group. Since the compound having the long-chain alkyl-based group is bound after the compound having the polyamino group is bound, it is possible to easily introduce the long-chain alkyl-based group, and reduce the positive electric charge on the surface of the polymer by improving the reaction efficiency.

For example, the biosensor chip according to the first embodiment can be produced by activating carboxyl groups on the polymer, causing a reaction of a polyamino compound with first part of the carboxyl groups, reactivating second part of the carboxyl groups which are not activated, causing a reaction of a compound having a long-chain alkyl-based group with the second part of the carboxyl groups, and finally performing acetylation. The acetylation is a process for attaching an acyl group onto the chip. The acyl group can be attached onto the chip by causing a reaction with the acyl group which is activated as a derivative such as acyl chloride or an acid anhydride. Alternatively, the biosensor chip according to the first embodiment can also be produced by reactivating the second part of the carboxyl groups which are not activated, and causing a direct reaction of a polyamino compound having an acyl group at an end of the polyamino compound, with the second part of the carboxyl groups reactivated as above.

The biosensor chip according to the second embodiment can be produced by activating the carboxyl group at the surface of the polymer, binding a reaction of a polyamino compound with the carboxyl group, and causing a reaction of a compound having a long-chain alkyl-based group with the polyamino compound.

The biosensor chip according to the third embodiment can be produced by activating the carboxyl group on the polymer, causing a reaction with a first polyamino compound and a reaction with a second polyamino compound having an acyl group at an end of the second polyamino compound, activating the first polyamino compound, and causing a reaction of a compound having a long-chain alkyl-based group with the first polyamino compound.

In the above processes for producing the biosensor chips according to the first to third embodiments, the polyamino compound may be a diaminoalkylene or a di(aminoalkyl) ether. Specifically, the polyamino compound is one of the following aliphatic diamines, aromatic diamines, and polyamines. The aliphatic diamines include ethylenediamine, tetraethylenediamine, octamethylenediamine, decamethylenediamine, piperazine, triethylenediamine, diethylenetriamine, triethylenetetramine, dihexamethylenetriamine, 1, 4-diaminocyclohexane, and the like. The aromatic diamines include paraphenylenediamine, methaphenylenediamine, paraxylylenediamine, methaxylylenediamine, 4,4'-diaminobiphenyl, 4,4'-diaminodiphenylmethane, 4,4'-diaminodiphenylketone, 4,4'-diaminodiphenylsulfone, and the like. The polyamines include diethylenetriamine, triethylenetetramine, tetraethylenepentamine, pentaethylenehexamine, spermidine, spermine, polyethyleneimine, and the like. Especially, from the viewpoint of water solubility and high reactivity in modification reaction, ethylenediamine, di(2-aminoethyl) ether, and di(3-aminopropyl) ether can be preferably used.

The compound having a long-chain alkyl-based group is preferably a fatty acid which contains an alkyl chain having 10 to 22 carbon atoms, and is more preferably a fatty acid which contains an alkyl chain having 12 to 18 carbon atoms. In some cases, one or more heteroatoms may exist between sequences of carbon atoms constituting the long-chain alkyl-based group, and/or one or more double bonds and/or one or more triple bonds may exist between single-bonded sequences of carbon atoms constituting the long-chain alkyl-based group. In addition, it is preferable that the long-chain alkyl-based group have a normal-chain structure (which is not branched). Specifically, preferable examples of the fatty acid are lauric acid, myristic acid, palmitic acid, stearic acid, arachidic acid, behenic acid, oleic acid, and the like. Among all, stearic acid and oleic acid are particularly preferable since stearic acid and oleic acid can realize both of simple modification reaction and absorption of lipids.

The polyamino compound having an acyl group at an end is a monoacylalkyldiamine, a monoacylalkylenediamine, a diacylalkyltriamine, or the like. Preferable examples of the polyamino compound having an acyl group at an end are monostearoylethylenediamine, monooleoylethylenediamine, monopalmitoylethylenediamine, monolauroylethylenediamine, monostearoylphenylenediamine, monooleoylphenylenediamine, monopalmitoylphenylenediamine, monolauroylphenylenediamine, 1,2,-oleoylpropanetriamine, monostearoyl(diaminoethyl ether), and monooleoyl(diaminoethyl ether). Among all, monostearoylethylenediamine, monooleoylethylenediamine, monostearoyl(diaminoethyl ether), and monooleoyl(diaminoethyl ether) are particularly preferable.

The above compounds can be attached onto the chip by causing a reaction with a reactive group which is activated by a process for activating a polymer as explained later.

### 1.3 Constituents of Biosensor Chip

Next, details of the substrate, the polymer, and the like, which constitute the biosensor chips according to present invention, are explained below.

### 1.3.1 Substrate and Metal Film

For example, in the case where the biosensor chip according to the present invention is to be used in a surface plasmon resonance biosensor, a substrate of a material transparent to laser light, such as optical glass (e.g., the type BK7 glass) or synthetic resin (e.g., polymethyl methacrylate, polyethylene terephthalate, polycarbonate, or a cycloolefin polymer) can be used as the substrate in the biosensor chip according to the present invention. It is preferable that the material of the substrate not be anisotropic to polarized light, and be superior in machinability or processibility.

In the above case, a metal film is arranged over the substrate. At this time, the metal film may be arranged in direct contact with the substrate, or over the substrate through another layer. The composition of the metal film is not specifically limited as long as the surface plasmon resonance can occur. However, it is preferable that the substrate be made of one or a combination of gold, silver, copper, platinum, palladium, and aluminum, and is particularly preferable that the substrate be made of gold. In addition, it is possible to arrange an interposing layer of chromium or the like between the substrate and the metal film.

Although the thickness of the metal film is not specifically limited, the thickness of the metal film is preferably 0.1 to 500 nm, and particularly preferably 1 to 200 nm. In the case where the thickness of the metal film exceeds 500 nm, it is impossible to sufficiently detect the surface plasmon resonance of a medium. In the case where the interposing layer of chromium or the like is arranged, the thickness of the interposing layer is preferably 0.1 to 10 nm.

The metal film may be formed by one of the conventional film-formation techniques such as sputtering, evaporation, ion plating, electroplating, and nonelectrolytic plating.

### 1.3.2 Polymer

The polymer (polymer film) is bound to the substrate through the metal film. The polymer film can be constituted by one or combination of hydrophilic polymers and hydrophobic polymers. However, it is preferable to bind a hydrophilic polymer from the viewpoint that bioactive substances can be three-dimensionally bound to the hydrophilic polymer. Although the polymer film may be bound to the substrate either directly or indirectly, it is preferable that the polymer film be formed on a self-assembled film.

The polymer in each of the biosensor chips according to the first to third embodiments has the carboxyl group. The carboxyl group may be originally contained in the polymer, or may be added to a polymer which does not originally contain the carboxyl group. The carboxyl group can be added by ozonization, plasma processing, hydrolysis of the polymer by alkali such as NaOH, binding of a linker having the carboxyl group, or another technique.

In addition, even in the biosensor chips in which the polymer contains another functional group having the negative electric charge such as the sulfonic acid (sulfo) group or the sulfinic group, instead of the carboxyl group, the negative electric charge at the surface of the polymer can also be cancelled by binding a polyamino group to the polymer. Therefore, such biosensor chips can also achieve the advantages similar to the present invention.

### 1.3.3 Self-assembled Film

The self-assembled film is an ultrathin film (such as a monomolecular film or an LB (Langmuir-Blodgett) film) which is formed into an ordered structure by a self-assembling mechanism possessed by the material of the film per se. The self-assembling mechanism enables formation of an ordered structure or pattern over a wide area under a non-equilibrium condition.

It is preferable that the self-assembled film be formed of a compound expressed by a constitutional formula X-R⁵-Y, where the X represents a group which can be bound to the metal film, R⁵ represents a divalent organic linking group, and Y represents a group which can be bound to hydrophilic polymers, NTA (nitrilotriacetic acid), and the like. Specifically, the group X is -SH, -SS, -SeH, -SeSe, -COSH, or the like, and the group Y is one of the functional groups of the hydroxy group, the hydroxycarbonyl group, alkoxy groups, and alkyl groups. In some cases, one or more heteroatoms may exist between sequences of carbon atoms constituting the divalent organic linking group R⁵. Preferably, the divalent organic linking group R⁵ is a normal (unbranched) organic chain structure suitable for desirably dense packing. In some cases, the divalent organic linking group R⁵ is a hydrocarbon chain having one or more double bonds and/or one or more triple bonds, or the hydrocarbon chain may be perfluorinated. It is preferable that the linking group R5 has a length corresponding to an alkyl chain containing 2 to 8 carbon atoms.

Further specifically, the self-assembled film can be preferably formed, on the metal film, of alkanethiols in the case where the metal film is formed of gold, alkylsilanes in the case where the metal film is formed of glass, and alcohols in the case where the metal film is formed of silicon. Specific examples of the alkanethiols which can be used for the self-assembled film are 7-carboxy-1-heptanethiol, 10-carboxyl-1-decanethiol, 4,4'-dithiodibutyric acid, and 11-hydroxy-1-undecanethiol, and 11-amino-1-undecanethiol. Specific examples of the alkylsilanes are aminopropyltrimethoxysilane, aminoethylaminotriethoxysilane, hydroxypropyltriethoxysilane, and the like. 1.3.4 Hydrophilic Polymer

The natural polymers such as dextran derivatives, starch derivatives, cellulose derivatives, and gelatines and the synthetic polymers such as polyvinyl alcohol, polyethylene glycol, polyvinylpyrrolidone, polyacrylamide derivatives, and polymethylvinyl ether are examples of the hydrophilic polymer.

In addition, synthetic polymers containing the carboxyl group and natural polymers containing the carboxyl group can be used as the polymer containing the carboxyl group. The synthetic polymers containing the carboxyl group include polyacrylic acid, polymethacrylic acid, and copolymers of polyacrylic acid and polymethacrylic acid. For example, the synthetic polymers containing the carboxyl group include the methacrylic acid coplymer, the acrylic acid coplymer, the itaconic acid coplymer, the crotonic acid coplymer, the maleic acid coplymer, the partially esterificated maleic acid coplymer, and the polymer produced by adding an acid anhydride to a polymer containing the hydroxy group, which are disclosed in Japanese Examined Patent Publication No. 59(1984)-044615 (corresponding to U.S. Patent No. 4,139,391), Japanese Examined Patent Publication No. 54 (1979)-034327 (corresponding to U.S. Patent No. 3,804,631), Japanese Examined Patent Publication No. 58(1983)-012577 (corresponding to U.S. Patent No. 3,930,865), Japanese Examined Patent Publication No. 54(1979)-025957 (corresponding to British Patent Publication No. 1 521 372), Japanese Unexamined Patent Publication No. 59(1984)-053836 (corresponding to U.S. Patent No. 4,687,727), and Japanese Unexamined Patent Publication No. 59(1984)-071048 (corresponding to U.S. Patent No. 4, 537, 855) .

The natural polymers containing the carboxyl group may be extracts from natural plants or products of microorganism fermentation, enzyme synthesis, and chemical synthesis. The natural polymers containing the carboxyl group include polysaccharides such as hyaluronic acid, chondroitin sulfate, heparin, dermatan sulfate, carboxymethylcellulose, carboxyethylcellulose, cellouronic acid, carboxymethyl chitin, carboxymethyl dexetran, and carboxymethyl starch, and polyamino acids such as polyglutaminic acid and polyaspartic acid. The polysaccharides containing the carboxyl group are commercially available. For example, carboxymethyl dexetran is available as the products CMD, CMD-L, and CMD-D40 from Meito Sangyo Co., Ltd, carboxymethylcellulose is available in the form of sodium carboxymethylcellulose from Wako Pure Chemical Industries, Limited, and alginic acid is available in the form of sodium alginate from Wako Pure Chemical Industries, Limited.

The polymer containing the carboxyl group used in the biosensor chip according to the present invention is preferably one of polysaccharides containing the carboxyl group, and more preferably carboxymethyl dexetran.

The molecular weight of the polymer containing the carboxyl group used in the biosensor chip according to the present invention is not specifically limited. However, the average molecular weight of the polymer containing the carboxyl group is preferably 1,000 to 5,000,000, more preferably 10,000 to 2,000,000, and further preferably 100, 000 to 1,000,000. When the average molecular weight of the polymer containing the carboxyl group is smaller than the range of 1,000 to 5, 000, 000, the immobilized amount of the bioactive substance becomes too small. On the other hand, when the average molecular weight of the polymer containing the carboxyl group is greater than the range of 1,000 to 5, 000, 000, high viscosity of the polymer solution makes the handling of the polymer solution difficult.

The hydrophilic polymer as described above may be bound to the substrate through the aforementioned self-assembled film or hydrophobic polymer, or may be formed directly on the substrate by use of a solution containing the polymer. In addition, the hydrophilic polymer may be bridged.

In the case where the hydrophobic polymer is used, preferable examples of the hydrophobic polymer are polyacrylic acid derivatives, polymethacrylic acid derivatives, polyethylene (PE), polypropylene (PP), polybutadiene, polymethylpentene, cycloolefin polymer, polystyrene (PS), acrylonitrile/butadiene/styrene copolymer (ABS), styrene/maleic anhydride copolymer/polyvinyl chloride (PVC), polyethylene terephthalate (PET), polyethylene naphthalate (PEN), nylon 6, nylon 66, cellulose acetate (TAC), polycarbonate (PC), modified polyphenylene ether (m-PPE), polyphenylene sulfide (PPS), polyether ketone (PEK), polyether ether ketone (PEEK), polysulfone (PSF), polyether sulfone (PES), polyphenylene sulfide (PPS), and liquid crystal polymers (LCP). Further, the above hydrophobic polymers can turn into a hydrophilic polymer when cationic groups are introduced into the hydrophobic polymer at a high rate.

The substrate can be coated with hydrophobic polymer by the conventional techniques such as spin coating, air-knife coating, bar coating, blade coating, slide coating, curtain coating, spraying, evaporation, casting, dipping (immersion), or the like.

### 1.3.5 Activation of Polymer

In the case where the hydrophilic polymer is used as the polymer containing the carboxyl group, and the substrate is coated with a self-assembled film, the polymer can be bound to the substrate by activating the carboxyl group. The polymer containing the carboxyl group can be preferably activated, for example, by using the following conventional techniques (1) to (3).
(1) The activation by use of 1-(3-dimethylaminopropyl)-3-ethylcarbodiimide (EDC) and N-hydroxysuccinimide (NHC), which are water-soluble carbodiimides
(2) The technique which is disclosed in Japanese Unexamined Patent Publication No. 2006-058071, and in which the carboxyl group is activated by use of one of the uronium salt, the phosphonium salt, and the triazine derivatives which has a specific structure
(3) The technique which is disclosed in Japanese Unexamined Patent Publication No. 2006-090781, and in which the carboxyl group is activated by processing using a triazine derivative or a salt of the triazine derivative, and subsequent processing using one of a nitrogen-containing hetero aromatic compound having the hydroxy group, a phenol derivative having an electron attracting group, and an aromatic compound having the thiol group

Although the polymer containing the activated carboxyl group can be bound to the substrate by causing a reaction of a solution of the polymer with the substrate, it is preferable to cause the reaction after a thin film is formed on the substrate by spin coating or the like.

After the carboxyl group in the polymer is activated as above, a reaction of a compound having the polyamino group with the polymer and a reaction of a compound having the long-chain alkyl-based group are caused. Thus, production of the biosensor chips according to the embodiments can be completed.

### 1.4 Bioactive Substance

The bioactive substance may be, for example, an immunoprotein, an enzyme, a microorganism, and a nucleic acid, a low-molecular organic compound, a nonimmune protein, an immunoglobulin-binding protein, a sugar-binding protein, a sugar chain which recognizes a type of sugar, a lipid, a fatty acid, a fatty acid ester, or a polypeptide or an oligopeptide having ligand-binding ability. The bioactive substance can be immobilized by application of or immersion in a solution containing the bioactive substance.

### 2. Biosensor

The scope of the biosensor of the present invention should be considered in the broadest meaning, and the biosensor of the present invention is a sensor which detects and measures an objective substance by converting an interaction between biomolecules into a signal such as an electric signal. Normally, the biosensor chip is constituted by a receptor part and a transducer part. The receptor part recognizes a chemical substance to be detected, and the transducer part coverts a physical or chemical change occurring in the receptor part into an electric signal. Living bodies contain such pairs of substances that the substances (in each pair) have an affinity for each other. Examples of such pairs are an enzyme and a substrate, an enzyme and a coenzyme, an antigen and an antibody, or a hormone and a receptor. When one of substances having an affinity for the other is fixed as a molecular recognizer to a substrate, the biosensor can selectively measure the other of the substances.

The biosensor chip according to the present invention can be used for detection and/or measurement of an interaction between a substance to be examined and a bioactive substance immobilized to the substrate of the biosensor chip. As mentioned before, the biosensor chip according to the present invention can be used in a biosensor performing one of the surface plasmon resonance (SPR) measurement, the quartz crystal microbalance (QCM) measurement, and the measurement using the functionalized surfaces of gold particles having various dimensions in the range from colloidal particles to ultrafine particles, and the like.

Preferably, the biosensor chip is used as a biosensor chip for surface plasmon resonance analysis, and a metal film is arranged on a transparent substrate. Although, generally, the biosensor chip for surface plasmon resonance analysis comprises a member containing a first part in which irradiation light propagates and reflects and a second part on which a bioactive substance is to be immobilized, the biosensor chip according to the present invention can be used as a member having the second part on which a bioactive substance is to be immobilized.

An example of a surface-plasmon-resonance measurement system which analyzes a property of a substance to be measured uses a system called the Kretschmann configuration. (See, for example, Japanese Unexamined Patent Publication No. 6(1994)-167443.) Basically, the above surface-plasmon-resonance measurement system is constituted by a dielectric block, a metal film, a light source, an optical system, and an optical detection means. The dielectric block is formed, for example, in the form of a prism. The metal film is formed on a face of the dielectric block so as to be in contact with a substance to be measured (such as a specimen solution) . The light source generates a light beam. The optical system can make the light beam inject onto the dielectric block at various incident angles so as to satisfy the total reflection at the interface between the dielectric block and the metal film. The optical detection means detects the surface-plasmon-resonance state, i.e., the attenuated total reflection (ATR)), by measuring the intensity of a portion of the light beam which is reflected at the above interface.

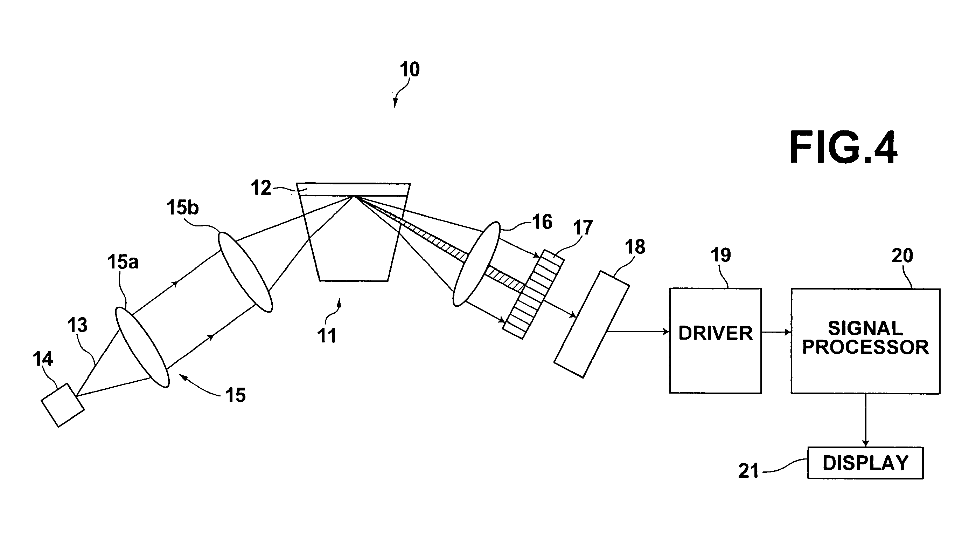

Hereinbelow, an embodiment of a sensor for surface plasmon resonance analysis according to the present invention is explained with reference to FIG. 4, which is a schematic side view illustrating a configuration of the sensor for surface plasmon resonance analysis, where the sensor comprises the biosensor chip according to the present invention. The sensor comprises a plurality of measurement units and a display unit 21. Each measurement unit comprises a biosensor chip 10, a laser-light source 14, an incident-beam optical system 15, a collimator lens 16, an optical detector 17, a differential-amplifier array 18, a driver 19, and a signal processing unit 20. The laser-light source 14 generates a light beam 13. The incident-beam optical system 15 makes the light beam 13 injected into the biosensor chip 10. The collimator lens 16 collimates the light beam 13 reflected by the biosensor chip 10, and outputs the reflected light beam 13 toward the optical detector 17. The optical detector 17 receives the reflected light beam 13 from the biosensor chip 10, and detects the intensity of the reflected light beam 13. The differential-amplifier array 18 is connected to the optical detector 17, and the driver 19 is connected to the differential-amplifier array 18. The signal processing unit 20 is realized by a computer system or the like, and connected to the driver 19. The signal processing unit 20 has a function of processing the signal outputted from the optical detector 17 through the differential-amplifier array 18 and the driver 19, and a function of correcting the sensitivity of the biosensor chip 10. That is, the signal processing unit 20 behaves as a sensitivity correction means as well as a signal processing means. The optical detector 17, the differential-amplifier array 18, the driver 19, and the signal processing unit 20 realize a measurement means for measuring the position of a dark line in the reflected light beam 13.

The biosensor chip 10 is constituted by a dielectric block 11 and a metal film 12. The dielectric block 11 has a shape produced by removing from a pyramid a portion containing the top of the pyramid and forming a recess in the base of the pyramid. The recess in the base has a function of holding a specimen solution, and the metal film 12 is formed on the recess of the base of the dielectric block 11. Although not shown in FIG. 4, the polymer is bound to the metal film 12, and the polyamino group and the long-chain alkyl-based group are directly or indirectly bound to the surface of the polymer.

In addition, the leakage-mode measurement system as reported, for example, in Bunko Kenkyu (Journal of the Spectroscopic Society of Japan, in Japanese), Vol. 47, No. 1, pp. 21-23 & 26-27, 1998 is another type of measurement system which also utilizes the attenuated total reflection (ATR). Specifically, the leakage-mode measurement system includes: a dielectric block having a prismatic shape; a cladding layer formed on a face of the dielectric block; an optical waveguide layer formed on the cladding layer so that the optical waveguide layer can be in contact with a specimen solution; a light source which generates a light beam; an optical system which makes the light beam injected into the dielectric block at various incident angles so that the light beam is totally reflected at the boundary between the dielectric block and the cladding layer; and a light detection unit which can detect the state in which the attenuated total reflection occurs (i.e., the state in which the propagation mode is excited) by measuring the intensity of the light beam totally reflected at the above boundary. The biosensor chip according to the present invention can also be used in such a leakage-mode measurement system.

Further, the biosensor chip according to the present invention can also be used in a biosensor which has a waveguide structure with a diffraction grating (and an additional layer in some cases), and detects change in the refractive index by use of the waveguide. The structures of the biosensor chips of this type are disclosed in, for example, Japanese Examined Patent Publication No. 6(1994)-027703, page 4, line 48 to page 14, line 15 and Figures 1 to 8 (and U.S. Patent No. 5,071,248, columns 3 to 13 and FIGS. 1 to 8) and U.S. Patent No. 6,829,073, column 6, line 31 to column 7, line 47 and FIGS. 9A and 9B. Furthermore, the biosensor chip according to the present invention can be used in a biosensor having another structure in which an array of grating-coupled waveguides are incorporated within wells of a microplate as disclosed in Japanese Unexamined Patent Publication No. 2007-501432 (corresponding to U.S. Patent No. 6, 985, 664) . That is, in the case where the grating-coupled waveguides are arrayed on the bottoms of the wells of the microplate, the screening of a medical or chemical substance can be performed with high throughput.

### 3. Evaluation of Examples

The present inventors have produced concrete examples of the biosensor chip according to the present invention as indicated below.

### 3.1 Concrete Example 1

The concrete example 1 of the biosensor chip according to the present invention has been produced as follows.

A 1:1 mixture of a water solution containing 2.8mM HODhbt (3,4-dihydro-3-hydroxy-4-oxo-1,2,3-benzotriazine) and a water solution containing 0.4M EDC (1-ethyl-3-(3-dimethylaminopropyl) ethylcarbodiimide hydrochloride, available from Dojindo Laboratories, Japan) is prepared, 100 ml of the mixture solution is placed in contact with the surface of a Biacore sensor chip CM-5 (which is the research grade, and available from GE Healthcare Bio-science KK, Japan), and a reaction with the mixture solution is continued for ten minutes at room temperature. Then, the chip is washed with water, and dried at room temperature in a vacuum drier for ten minutes. Subsequently, 100 ml of 1, 2-bis (2-aminoethoxy)ethane is placed in contact with the surface of the chip, and a reaction with 1,2-bis(2-aminoethoxy)ethane is continued for ten minutes at room temperature. Then, the chip is washed with water. Thereafter, a solution of 1.0 g of stearic acid (available from Signa-Aldrich Japan K.K.) and 0.1 g of Tween 20 (available from Tokyo Chemical Industries Co., Ltd.) in 1.0 ml of DMF (dimethylformamide) is prepared, and 0.5 ml of a water solution of 0.1M N-hydroxysuccinic acid imide (available from Dojindo Laboratories, Japan) and 0.5 ml of a water solution of 0.4M EDC are added to and mixed in 0.5 ml of the DMF solution of Tween 20 and stearic acid and a reaction with the mixed solution is continued for ten minutes at room temperature. Then, the chip is washed with DMF, and thereafter with water. Thus, the concrete example 1 of the biosensor chip according to the present invention has been obtained.

### 3.2 Concrete Example 2

The concrete example 2 of the biosensor chip according to the present invention has been produced in a manner similar to the concrete example 1 except that oleic acid is used instead of stearic acid.

### 3.3 Concrete Example 3

The concrete example 2 of the biosensor chip according to the present invention has been produced as follows.

A 1:1 (v/v) mixture of a water solution containing 0.1mM NHS (N-hydroxysuccinamide) and a water solution containing 0.4M EDC is prepared, and 100 ml of the mixture solution is placed in contact with the surface of a Biacore sensor chip CM-5 (research grade). Then, the chip is washed with water, and a reaction with a solution of 0.1 mg of oleylamine in 1 ml of DMF is continued for ten minutes at room temperature. Subsequently, the chip is washed with water and dried. Thereafter, a 1:1 (v/v) mixture of a water solution containing 2.8mM HODhbt and a water solution containing 0.4M WSC (water-soluble carbodiimide) is prepared, 100 ml of the mixture solution is placed in contact with the surface of the chip, and a reaction with the mixture solution of HODhbt and WSC is caused. Then, the chip is washed with water, and dried at room temperature in a vacuum drier for ten minutes. Next, 100 ml of 1, 2-bis (2-aminoethoxy)ethane is placed in contact with the surface of the chip, and a reaction with 1,2-bis(2-aminoethoxy)ethane is continued for ten minutes at room temperature. Then, the chip is washed with water. Further, a solution of 0.1 mg of acetyl chloride in 1.0 ml of DMF is prepared, and a reaction of with the DMF solution of acetyl chloride is caused, and the chip is left unmoved at room temperature for one hour. Then, the chip is washed with DMF, and thereafter with water. Thus, the concrete example 3 of the biosensor chip according to the present invention has been obtained.

### 3.4 Concrete Example 4

The concrete example 4 of the biosensor chip according to the present invention has been produced as follows.

A 1:1 mixture of a water solution containing 2.8mM HODhbt and a water solution containing 0.4M EDC is prepared, 100 ml of the mixture solution is placed in contact with the surface of a Biacore sensor chip CM-5 (research grade), and a reaction with the mixture solution is continued for ten minutes at room temperature. Then, the chip is washed with water, and dried at room temperature in a vacuum drier for ten minutes. Subsequently, 100 ml of 1, 2-bis (2-aminoethoxy) ethane is placed in contact with the surface of the chip, and a reaction with 1,2-bis(2-aminoethoxy)ethane is continued for ten minutes at room temperature. Then, the chip is washed with water. Thereafter, a solution of 1.0 g of stearic acid in 1.0 ml of DMF is prepared, and 0.5 ml of a water solution of 0.1M N-hydroxysuccinic acid imide and 0.5 ml of a water solution of 0.4M EDC are added to and mixed in the DMF solution of stearic acid, and a reaction with the mixed solution is continued for ten minutes at room temperature. Then, the chip is washed with DMF, and thereafter with water. Further, a solution of 0.1 mg of acetyl chloride in 1.0 ml of DMF is prepared, and a reaction of with the DMF solution of acetyl chloride is caused, and the chip is left unmoved at room temperature for one hour. Then, the chip is washed with DMF, and thereafter with water. Thus, the concrete example 4 of the biosensor chip according to the present invention has been obtained.

### 3.5 Measurement of Sensorgram

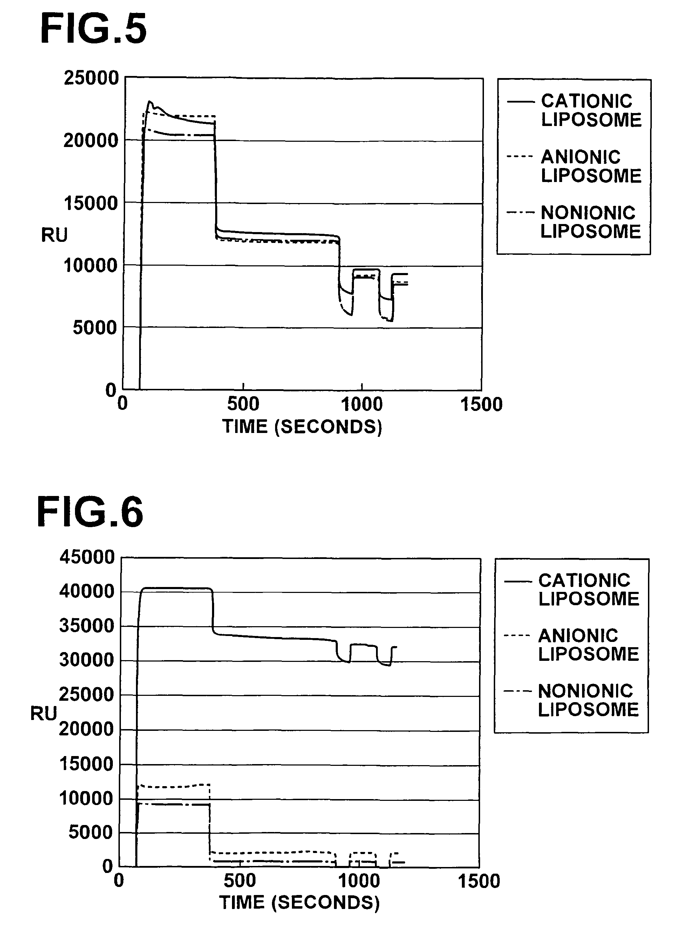

The present inventors have measured the absorbed amounts of three types of liposomes (cationic, anionic, and nonionic liposomes) by the biosensor chip as the concrete example 1 and the Biacore sensor chip L1 (which is available from GE Healthcare Bio-science KK, Japan, and used as a comparison example), where the liposomes used in the measurement are ones of the liposomes COATSOME which are available from NOF Corporation. In the Biacore sensor chip L1, dextran arranged on a substrate is modified with a long-chain alkane. (COATSOME is a registered trademark of NOF Corporation.) In the measurement, each of the biosensor chip as the concrete example 1 and the Biacore sensor chip L1 as the comparison example is set on the surface plasmon resonance system Biacore 3000 (available from GE Healthcare Bio-science KK, Japan). FIGS. 5 and 6 show the sensorgrams which have been obtained by the above measurement.

### 3.6 Measurement of Ability to Prevent Pollution

The present inventors have measured the ability to prevent pollution of the biosensor chips as the concrete examples 1 to 4 and the Biacore sensor chip L1 as the comparison example on the basis of absorption of methylene blue. In the measurement, each of the biosensor chips as the concrete examples 1 to 4 and the Biacore sensor chip L1 as the comparison example is set on the surface plasmon resonance system Biacore 3000. The results of the measurement, as well as the amounts of absorption of the cationic, anionic, and nonionic liposomes, are indicated in Table 1, where absorption of methylene blue from 0 to 2000 RU (resonance unit) is indicated by a blank circle, and absorption of methylene blue greater than 2000 RU is indicated by a cross.

**Table 1**

| | Absorbed Amount of Liposome | | | Ability to Protect pollution |
|---|---|---|---|---|
| | Cationic | Anionic | Nonionic | |
| Concrete Example 1 | 8707 | 7636 | 7644 | ○ |
| Concrete Example 2 | 9358 | 8757 | 8530 | ○ |
| Concrete Example 3 | 11688 | 9265 | 9852 | ○ |
| Concrete Example 4 | 8690 | 10067 | 10447 | ○ |
| Comparison Example | 31860 | 2062 | 794 | X |

As understood from FIGS. 5 and 6 and Table 1, the amounts of absorption of the anionic and nonionic liposomes by the sensor chip as the comparison example are extremely small, and the amounts of absorption greatly vary depending on the electric charge. On the other hand, the biosensor chips as the concrete examples 1 to 4 absorb all of the cationic, anionic, and nonionic liposomes. That is, the biosensor chip according to the present invention can absorb liposomes regardlessly of the electric charge. Therefore, the biosensor chip according to the present invention has great versatility. In addition, as indicated in Table 1, the amount of absorption of low-molecular-weight compounds by the biosensor chip according to the present invention is small. That is, the biosensor chip according to the present invention exhibits high ability to protect pollution.

## Claims

1. A biosensor chip comprising,
a substrate, and
a polymer having an anionic functional group and being arranged on a surface of said substrate;
**characterized in** further comprising,
a first polyamino group which is directly or indirectly bound to said anionic functional group at a surface of said polymer, and
a long-chain alkyl-based group which is directly or indirectly bound to said anionic functional group at said surface of said polymer.

2. A biosensor chip according to claim 1, wherein said first polyamino group has an acyl group at an end of the first polyamino group.

3. A biosensor chip according to claim 1, wherein said long-chain alkyl-based group is bound to said first polyamino group.

4. A biosensor chip according to claim 3, further comprising a second polyamino group which is directly or indirectly bound to said polymer.

5. A biosensor chip according to either of claims 1 to 4, wherein said first polyamino group is a diaminoalkylene group or a di(aminoalkyl) ether group.

6. A biosensor chip according to either of claims 1 to 5, wherein said long-chain alkyl-based group is an alkyl-based group having 10 to 22 carbon atoms.

7. A biosensor chip according to either of claims 1 to 6, wherein said polymer is carboxymethyl dextran.

8. A biosensor chip according to either of claims 1 to 7, wherein said polymer is arranged on said substrate through a metal film.

9. A biosensor chip according to claim 8, wherein said metal film is composed of at least one of metals of gold, silver, copper, platinum, and aluminum.

10. A biosensor chip according to either of claims 1 to 9, wherein said anionic functional group is a carboxyl group.

11. A process for producing a biosensor chip, comprising the steps of:
(a) activating an anionic functional group in a polymer arranged on a surface of a substrate; and
(b) binding a first compound containing a polyamino group to said anionic functional group, and thereafter binding a second compound containing a long-chain alkyl-based group to said anionic functional group.

12. A process according to either of claim 11, wherein said anionic functional group is a carboxyl group.

13. A sensor for surface plasmon resonance analysis comprising said biosensor chip according to either of claims 1 to 10.
